(19) Europäisches Patentamt
European Patent Office
Office européen des brevets

(11) Publication number: **0 100 459 B1**

(12) **EUROPEAN PATENT SPECIFICATION**

(45) Date of publication of patent specification: **11.11.92**

(51) Int. Cl.[5]: **A61K 47/10**, A61K 7/48

(21) Application number: **83106668.3**

(22) Date of filing: **07.07.83**

# (54) An aqueous liquid containing a fat-soluble substance.

(30) Priority: **07.07.82 JP 116919/82**

(43) Date of publication of application:
**15.02.84 Bulletin 84/07**

(45) Publication of the grant of the patent:
**11.11.92 Bulletin 92/46**

(84) Designated Contracting States:
**AT BE CH DE FR GB IT LI NL SE**

(56) References cited:
**EP-A- 0 019 301**
**EP-A- 0 083 108**

**CHEMICAL ABSTRACTS, vol. 83, no.16, 20 October 1975, page 350, abstract 136886q (COLUMBUS OHIO, US).**

**CHEMICAL ABSTRACTS, vol. 89, no.12, 18 September 1978, page 307, abstract 95002b (COLUMBUS, OHIO, US).**

**JP-A-53-56315**

(73) Proprietor: **Eisai Co., Ltd.**
**6-10, Koishikawa 4-chome Bunkyo-ku**
**Tokyo 112(JP)**

(72) Inventor: **Shigemitsu, Osawa**
**2286-12, Suehiro-cho**
**Honjyo-shi Saitama Prefecture(JP)**
Inventor: **Iwao, Amada**
**644-1, Naka**
**Fujioka-shi Gumma Prefecture(JP)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to an aqueous liquid containing as essential components a fat-soluble substance selected from vitamin A, vitamin E, vitamin D, vitamin K, ubidecarenone, derivatives of these substances, bisabolol, salicylic acid esters, squalane, isopropyl myristate and vegetable oils, furthermore lecithin and at least one alcohol selected from ethanol and isopropanol.

Examples have heretofore been known in which large amounts of nonionic surface-active agents are used in order to clearly dissolve or emulsify fat-soluble substances in water It is well known, however, that by any of various administration methods, nonionic surface-active agents cause some undesirable trouble to humans. In view of this, techniques were developed for using lecithin, a natural substance with relatively high safety, instead of synthetic surfactants, as seen, for example, in Japanese Patent Applications Laid-Open Nos. 62010/1980, 147228/1980, 38314/1980, 83912/1977, 83911/1977, 50124/1974 and 126821/1975. With these techniques, exertion of a strong mechanical stress is required in order to supplement the weak emulsifying power of lecithin. Furthermore, since the average particle diameter of particles in the resulting emulsion is as large as 0.5 $\mu$m or more and the particle size distribution is broad, the stability of the emulsion with the lapse of time is not sufficient. With this background, some of the present inventors studied a system composed of a fat-soluble substance, lecithin and ethanol, and discovered a system composed of 1 part by weight of a fat-soluble substance, 0.1 to 10 parts by weight of lecithin and 1 to 50 parts by weight of ethanol, which can be emulsified by addition of water. An invention based on this discovery was applied for a patent in Japanese Patent Application Laid-Open No. 56315/1978 (= JP-A-53-56315A).

Subsequently, the present inventors studied a system consisting essentially of a fat-soluble substance, lecithin and an alcohol, which can be converted to a stable emulsion or solution by a simple convenient method. It is necessary that the concentration of alcohol in the emulsion or solution should be as high as 20 to 50% for example, in order to use the emulsion or solution as a lotion, a skin lotion or a milky lotion. The present inventors made investigations in order to obtain such a system. Generally, the addition of a large amount of an alcohol to an emulsion or solution of a fat-soluble substance prepared by emulsifying or dissolving it with the aid of a surface-active substance such as lecithin adversely affects the emulsion or solution, and such an addition, without adverse effects, has been considered to be a difficult technique. For example, BBA 298 (1973) 1015-1019 reported that when an ethanol solution of lecithin was added to a 0.16M aqueous solution of KCl, the concentration of ethanol in the resulting solution was 7.5% at the highest. In order, therefore, to prepare a stable emulsified or solubilized system by adding a fat-soluble substance and lecithin to an aqueous solution of an alcohol at a high concentration, it is considered necessary to set certain numerical limitations on the proportions of these ingredients to be blended.

The present inventors studied systems containing various components starting with a system containing an alcohol at a high concentration, in view of the need to use an emulsified or solubilized liquid containing a fat-soluble substance, lecithin and an alcohol as essential components as a lotion, a skin lotion or a hair tonic. It was first found that besides ethanol, isopropanol can be used as the alcohol, and ethanol can be incorporated at a concentration of up to 50% by volume. It was also found that when the alcohol is ethanol to be incorporated at a concentration of not more than 50 V/V% of the aqueous liquid and when it is added in an amount of less than 50 parts by weight per part by weight of the fat-soluble substance, the amount of lecithin should be less than 0.1 part by weight per part by weight of the fat-soluble substance; when ethanol is incorporated in an amount of 50 parts by weight or more per part by weight of the fat-soluble substance, the amount of lecithin should be not more than 50 parts by weight per part by weight of the fat-soluble substance; and when the alcohol is isopropanol in a concentration of 10 to 30 V/V% in the aqueous liquid, the amount of lecithin should be not more than 50 parts by weight per part by weight of the fat-soluble substance. The foregoing findings have led to the present invention.

It is an object of this invention to provide a stable aqueous liquid containing a fat-soluble substance, lecithin and ethanol or isopropanol as essential components.

This object is solved by an aqueous liquid containing as essential components a fat-soluble substance selected from vitamin A, vitamin E, vitamin D, vitamin K, ubidecarenone, derivatives of these substances, bisabolol, salicylic acid esters, squalane, isopropyl myristate and vegetable oils, furthermore lecithin and at least one alcohol selected from ethanol and isopropanol

a) the volume of ethanol being not more than 50 V/V% of the aqueous liquid, and the amount of lecithin being less than 0.1 part by weight per part by weight of the fat-soluble substance, when ethanol is used as the alcohol in an amount of less than 50 parts by weight per part by weight of the fat-soluble substance, or

b) the volume of ethanol being not more than 50 V/V% of the aqueous liquid, and the amount of lecithin

being not more than 50 parts by weight per part by weight of the fat-soluble substance, when ethanol is used as the alcohol in an amount of at least 50 parts by weight per part by weight of the fat-soluble substance, or

c) the volume of isopropanol being 10 to 30 V/V% of the aqueous liquid and the amount of lecithin being not more than 50 parts by weight per part by weight of the fat-soluble substance.

The aqueous liquid containing a fat-soluble substance in accordance with this invention denotes an emulsion or solution. The aqueous liquid contains a fat-soluble substance, lecithin as well as ethanol or iso-propanol as essential components.

The fat-soluble substance used in this invention denotes one or more of fat-soluble substances for medicines, cosmetics and foodstuffs, which are soluble in ethanol or iso-propanol. The fat-soluble substance is selected from vitamins A, E, D and K, ubidecarenone and derivatives of these substances, coenzymes such as $CoQ_8$ and $CoQ_{10}$, salicyclic acid esters, methyl salicylate, monoglycol salicylate, bisabolol, $\alpha$-bisabolol, squalane and isopropyl myristate and vegetable oils. The vegetable oils denote for instance sesame oil, soybean oil and olive oil.

Lecithin used in this invention denotes a phospholipid-containing substance extracted from egg yolk or from a vegetable oil and optionally purified, or a hydrogenation product thereof. It also denotes synthetic glycerophosphoric acid esters of the following general formula, or mixtures thereof.

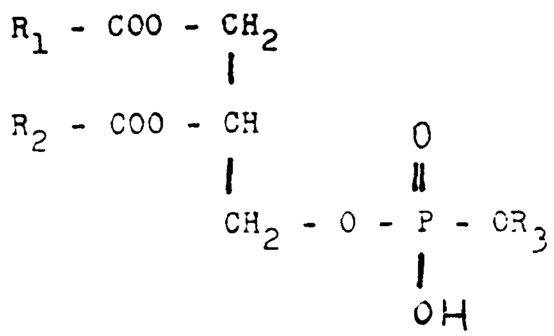

In the formula, $R_1$ and $R_2$ each represent a hydrogen atom or a saturated or unsaturated hydrocarbon having 8 to 20 carbon atoms, and $R_3$ represents for instance a hydrogen atom, choline, ethanolamine, serine, inositol, glycerol.

Ethanol and iso-propanol are used either singly or in combination. When they are used in combination, the ratio between the two may be any desired one. The volume of ethanol is not more than 50% of the aqueous liquid of this invention containing a fat-soluble substance. The percentage here expresses V/V%.

In the aqueous liquid of this invention, the proportions of the fat-soluble substance, lecithin and the alcohol are limited. They differ whether the alcohol used is ethanol or iso-propanol. When ethanol is used in an amount of less than 50 parts by weight per part by weight of the fat-soluble substance, the amount of lecithin is less than 0.1 part by weight. When ethanol is used in an mount of at least 50 parts by weight per part by weight of the fat-soluble substance, the amount of lecithin is not more than 50 parts by weight. When iso-propanol or the ethanol-isopropanol mixture is used as the alcohol, the amount of lecithin is not more than 50 parts by weight.

The aqueous liquid of this invention may optionally contain for instance emulsifiers, isotonizing agent, buffers, dissolution aids, corrigents, antiseptics and stabilizers in addition to the essential components, as shown in Examples given hereinafter. The addition of these optional substances is free and does not limit the present invention.

The aqueous liquid of this invention is used for medicines, cosmetics and foodstuffs. Specific forms of the aqueous liquid include, for example, an injectable preparation, an orally administrable liquid preparation, a lotion, a hair tonic and a drink preparation.

The aqueous liquid of this invention may be prepared by general ordinary methods for the production of aqueous liquids. For example, the fat-soluble substance and lecithin are dissolved in ethanol or iso-propanol. The solution is added to an aqueous solution containing optional components, and the mixture is stirred in an ordinary manner. The stirring may, for example, be carried out by applying a pressurizing treatment or an ultrasonication treatment.

The following Experimental Examples illustrate the effects of the present invention.

Experimental Example 1

Sample and method:-

Tocopherol acetate (0.02 g) and purified hydrogenated egg yolk lecithin (0.3 g) were dissolved in ethanol (0.1 - 60 ml) having each the concentrations shown in Table 1. The solution was heated to about 40°C, and purified water warmed at about 35°C was injected under pressure into the solution with stirring to form an emulsion or solution. Purified water was added to adjust the total amount of the emulsion or solution to 100 ml and thus provide a sample. In these samples containing 1 part by weight of tocopherol acetate, 15 parts by weight of purified hydrogenated lecithin and 4 to 2400 parts by weight of ethanol were prepared, and their appearances and percent transmittances at 640 nm ($T_{640}$) were determined upon preparation and after storage for 30 days at 5°C and 45°C respectively.

Results:-

The results are summarised in Table 1. It is seen from Table 1 that stable aqueous liquids can be obtained when the concentration of ethanol is not more than 50 V/V %, particularly not more than 40 V/V %.

Table 1

| Concentration of ethanol (V/V %) | Upon preparation | | After storage at 45 °C for 30 days | | After storage at 5 °C for 30 days | | Stability |
|---|---|---|---|---|---|---|---|
| | Appearance | $T_{640}$ (%) | Appearance | $T_{640}$ (%) | Appearance | $T_{640}$ (%) | |
| 0.1 | Slightly emulsified | 81.0 | Same as left | 76.5 | Same as left | 75.5 | Nearly good |
| 0.5 | Somewhat emulsified | 46.5 | Same as left | 42.7 | Same as left | 40.0 | " |
| 1.0 | Emulsified | 19.0 | Same as left | 16.2 | Same as left | 16.6 | " |
| 3.0 | Slightly emulsified | 74.0 | Same as left | 75.5 | Same as left | 72.5 | Good |
| 5.0 | Slightly emulsified | 84.5 | Same as left | 85.0 | Same as left | 84.5 | Good |
| 10.0 | Almost clear | 92.0 | Same as left | 93.5 | Same as left | 92.8 | Good |
| 20.0 | Almost clear | 92.5 | Same as left | 93.5 | Same as left | 92.5 | Good |
| 30.0 | Almost clear | 93.0 | Same as left | 89.5 | Same as left | 92.2 | Good |
| 40.0 | Somewhat emulsified | 54.5 | Same as left | 32.5 | Same as left | 56.0 | Nearly good |
| 50.0 | Emulsified; lecithin partly precipitated | 12.5 | Same as left | 3.4 | Same as left | 4.2 | Somewhat poor |

The concentration of ethanol in the table denotes the concentration (V/V %) of ethanol in the aqueous liquid.

Experimental Example 2

Sample and method:-

Squalane (0.5 g) and purified hydrogenated egg yolk lecithin (0.001 to 0.35 g) in the weight parts shown

in Table 2 were dissolved in 20 ml of ethanol. The solution was heated to about 40°C. The solution was injected under pressure into purified water warmed at about 35°C with stirring to form an emulsion. The purified water was added to the emulsion to adjust its total amount to 100 ml and thus provide a sample. The resulting samples contained 1 part by weight of squalane, 0.002 to 0.1 part by weight of purified hydrogenated egg yolk lecithin and 32 parts by weight of ethanol, and the concentration of ethanol in each of the samples was 20 V/V %. The samples were left to stand for 30 days at 45°C, 5°C and room temperature, and changes in appearance and percent transmittances at 640 nm, $T_{640}$(%), were determined.

Results:-

Table 2 shows the results obtained with samples left to stand at room temperature for 30 days. No separation was observed in any of the samples after standing for 30 days at 45°C and 5°C, respectively. It is thus seen that when 32 parts by weight of ethanol is used per part by weight of squalane, the amount of purified hydrogenated egg yolk lecithin may be not more than 0.1 part by weight.

Table 2

| Parts by weight of purified hydrogenated egg yolk lecithin | After standing for 30 days at room temperature | | Stability |
|---|---|---|---|
| | Appearance | $T_{640}$(%) | |
| 0.002 | Uniform emulsion | 52.2 | Nearly good |
| 0.004 | Uniform emulsion | 56.0 | Good |
| 0.01 | Uniform, slightly bluish white emulsion | 68.0 | Good |
| 0.02 | Uniform, slightly bluish white emulsion | 75.0 | Good |
| 0.1 | Uniform emulsion | 15.0 | Nearly good |

In the table, the parts by weight of purified hydrogenated egg yolk lecithin are per part by weight of squalane.

Experimental Example 3

Sample and method:-

Squalane (0.1 g) and purified soybean lecithin (0.03 g) were dissolved in iso-propanol (0.5 to 60 ml) having each of the concentrations shown in Table 3. Then, samples were prepared by the same procedure as in Experimental Example 1. Each of the samples contained 1 part by weight of squalane, 0.3 part by weight of purified soybean lecithin and 5 to 600 parts by weight of iso-propanol. By the same method as in Experimental Example 1, the stabilities of the samples were determined.

Results:-

The results are summaried in Table 3. It is seen from Table 3 that stable aqueous liquids can be obtained when the concentration of iso-propanol is 10 to 30 V/V% of the aqueous liquid according to the invention.

Table 3

| Concentration of isopropanol (V/V %) | Upon preparation | | After storage at 45°C for 30 days | | After storage at 5°C for 30 days | | Stability |
|---|---|---|---|---|---|---|---|
| | Appearance | $T_{640}$(%) | Appearance | $T_{640}$(%) | Appearance | $T_{640}$(%) | |
| 10.0 | Almost clear | 92.5 | Same as left | 93.2 | Same as left | 91.5 | Good |
| 20.0 | Clear | 99.3 | Same as left | 98.8 | Same as left | 99.2 | Good |
| 30.0 | Clear | 99.1 | Same as left | 98.9 | Same as left | 99.1 | Good |

In the table, the concentration of isopropanol denotes the concentration (V/V %) of isopropanol in the aqueous liquid.

Experimental Example 4

Squalane (0.1 g) and purified hydrogenated egg yolK lecithin (0.001 to 6 g) corresponding to the weight parts indicated in Table 4 were dissolved in 20 ml of ethanol, followed by the same procedure as in Experimental Example 2, to prepare samples. Each of the samples contained 1 part by weight of squalane,

0.01 to 60 parts by weight of purified hydrogenated egg yolk lecithin and 200 parts by weight of ethanol, and the concentration of ethanol in each of the samples was 20 V/V %. The samples were left to stand for 30 days at room temperatures, and changes in appearance and percent transmittances at 640 nm, $T_{640}$(%), were determined.

Results:-

The results are shown in Table 4. It is seen from Table 4 that when 200 parts by weight of ethanol is used per part by weight of squalane, the amount of purified hydrogenated egg yolk lecithin may be not more than 50 parts by weight.

Table 4

| Parts by weight of purified hydrogenated egg yolk lecithin | After standing at room temperature for 30 days | | Stability |
|---|---|---|---|
| | Appearance | $T_{640}$ (%) | |
| 0.01 | Clear solution | 99.5 | Good |
| 0.02 | Clear solution | 97.5 | Good |
| 0.05 | Clear solution | 95.1 | Good |
| 0.1 | Clear solution | 96.7 | Good |
| 0.2 | Clear solution | 97.8 | Good |
| 0.5 | Clear solution | 98.8 | Good |
| 1.0 | Clear solution | 98.1 | Good |
| 2.0 | Nearly clear solution | 90.1 | Good |
| 10.0 | Uniform emulsion | 66.8 | Good |
| 30.0 | Uniform emulsion | 31.9 | Good |
| 50.0 | Nearly uniform emulsion | 6.3 | Good |

The parts by weight of the purified hydrogenated egg yolk lecithin in the table are per part by weight of squalane.

The following Examples illustrate the present invention more specifically.

Example 1

| Ubidecarenone injectable preparation:- | |
|---|---|
| Ubidecarenone | 1.0 g |
| Purified egg yolk lecithin | 0.4 g |
| Ethanol | 65.0 ml |
| Macrogol 400 | 50.0 g |
| Sorbitol | 45.0 g |
| Distilled water for injection | ad. 1,000 ml |

Ubidecarenone and purified egg yolk lecithin were dissolved in ethanol, and then Macrogol 400 (Macrogol 400 is a general name for "polyethylene glycol". The figure of "400" represents the mean polymerization degree). was dissolved in the solution. The resulting solution (solution I) was heated to about 45°C.

Sorbitol was dissolved in distilled water for injection, and the resulting solution (solution II) was heated to about 40°C.

The solution I prepared earlier was injected into the stirred solution II to prepare a solution of

ubidecarenone. The solution was cooled to room temperature and filtered through a membrane having a pore diameter of 0.22 μm. The filtrate was dividedly put in ampoules. The ampoules were sealed up by melting and heat-sterilized at 115°C for 30 minutes to form ubidecarenone injectable preparations. The injectable preparations were clear in appearance and stable.

Example 2

| Menatetrenone Injectable preparation:- | |
| --- | --- |
| Menatetrenone | 1.0 g |
| Purified sesame oil | 0.4 g |
| Purified egg yolk lecithin | 0.4 g |
| Purified soybean lecithin | 0.2 g |
| Ethanol | 90.0 ml |
| Macrogol 400 | 50.0 g |
| Glucose | 50.0 g |
| Distilled water for injection | ad. 1,000 ml |

Menatetrenone, purified sesame oil, purified egg yolk lecithin and purified soybean lecithin were dissolved in a mixture of ethanol and Macrogol 400, and the solution was maintained at about 50°C (solution I).

Glucose was dissolved in distilled water for injection, and the solution was maintained at about 45°C (solution II).

Thereafter, a menatetrenone injectable preparation was formed from the solution I and the solution II, by the same procedure as in Example 1. The injectable preparation obtained was nearly clear in appearance and remained stable with time.

Example 3

| Skin lotion:- | |
| --- | --- |
| Purified hydrogenated soybean lecithin | 2.0 g |
| Tocopherol acetate | 1.0 g |
| Squalane | 1.0 g |
| Ethanol | 140.0 ml |
| Glycerol | 40.0 g |
| Sorbitol | 40.0 g |
| Perfume | 0.7 g |
| Purified water | ad. 1,000 ml |

Purified hydrogenated soybean lecithin, tocopherol acetate, the perfume and squalane were dissolved in ethanol, and the solution was maintained at about 45°C (solution I).

Separately, glycerol and sorbitol were dissolved in purified water, and the solution was maintained at about 40°C (solution II).

Then, the solution I was injected into the stirred solution II to form a skin lotion which was uniform and bluish white with slight turbidity and stable.

Example 4

| Medicated hair tonic:- | |
|---|---|
| dℓ-α-tocopherol nicotinate | 1.0 g |
| Squalane | 2.0 g |
| Irgasan DP-300® | 0.5 g |
| Cholesterol | 0.2 g |
| ℓ-Menthol | 0.5 g |
| Purified hydrogenated egg yolk lecithin | 1.0 g |
| Ethanol | 350 ml |
| Propylene glycol | 60.0 g |
| Perfume | 2.0 g |
| Purified water | ad. 1,000 ml |

dℓ-α-Tocopherol nicotinate, Irgasan DP-300®, cholesterol, ℓ-menthol, the perfume and purified hydrogenated egg yolk lecithin were dissolved in a mixture of ethanol and propylene glycol, and the solution was maintained at about 45°C (solution I).

Purified water was heated and maintained at about 40°C (liquid II).

Thereafter, a hair tonic having a uniform, clear, bluish white appearance was obtained from the solution I and the liquid II, by the same procedure as in Example 3. The resulting hair tonic remained stable with time.

Example 5

| Drink preparation:- | |
|---|---|
| Tocopherol acetate | 1.0 g |
| Purified soybean lecithin | 0.02 g |
| Hydrogenated egg yolk lecithin | 0.03 g |
| Ascorbic acid | 1.0 g |
| Sodium citrate | 0.3 g |
| Sorbitol | 50.0 g |
| Sucrose | 80.0 g |
| Flavor | 0.2 g |
| Ethyl paraben | 0.1 g |
| Ethanol | 10.0 ml |
| Propylene glycol | 25.0 g |
| Purified water | ad. 1,000 ml |

Tocopherol acetate, purified soybean lecithin, hydrogenated egg yolk lecithin and the flavor were dissolved in ethanol, and the solution was maintained at about 40°C(solution I). The solution I was injected under pressure into purified water warmed at about 35°C by the same procedure as in Experimental Example 1 to give a uniform, bluish white, clear solution.

To the solution was added propylene glycol having dissolved therein ascorbic acid, sodium citrate, sorbitol, sucrose and ethyl paraben. They were dissolved uniformly to obtain a liquid preparation for oral administration. This solution showed a uniform, bluish white, clear appearance and was stable.

Example 6

| Hair tonic:- | |
|---|---|
| Tocopherol acetate | 1.0 g |
| Squalane | 2.0 g |
| Hinokitiol | 0.5 g |
| Cholesterol | 0.1 g |
| Benzyl nicotinate | 0.05 g |
| ℓ-Menthol | 0.5 g |
| Purified soybean lecithin | 4.0 g |
| iso-Propanol | 50.0 ml |
| Ethanol | 300.0 ml |
| Glycerol | 60.0 g |
| Perfume | 2.0 g |
| Purified water | ad. 1,000 ml |

Tocopherol acetate, squalane, hinokitiol, cholesterol, benzyl nicotinate, ℓ-menthol, the perfume and purified soybean lecithin were dissolved in a mixture of ethanol and isopropanol, followed by adding glycerol thereto. The solution was maintained at about 45°C (solution I).

Purified water was heated and maintained at about 40°C (solution II).

Thereafter, by the same procedure as in the preparation of the skin lotion in Example 3, a hair tonic having a uniform, bluish white, clear appearance was obtained. The resulting hair tonic remained stable with time.

## Claims

### Claims for the following Contracting States : BE, CH, DE, FR, GB, IT, LI, NL, SE

**1.** An aqueous liquid containing as essential components a fat-soluble substance selected from vitamin A, vitamin E, vitamin D, vitamin K, ubidecarenone, derivatives of these substances, bisabolol, salicylic acid esters, squalane, isopropyl myristate and vegetable oils, furthermore lecithin and at least one alcohol selected from ethanol and isopropanol

a) the volume of ethanol being not more than 50 V/V% of the aqueous liquid, and the amount of lecithin being less than 0.1 part by weight per part by weight of the fat-soluble substance, when ethanol is used as the alcohol in an amount of less than 50 parts by weight per part by weight of the fat-soluble substance, or

b) the volume of ethanol being not more than 50 V/V% of the aqueous liquid, and the amount of lecithin being not more than 50 parts by weight per part by weight of the fat-soluble substance, when ethanol is used as the alcohol in an amount of at least 50 parts by weight per part by weight of the fat-soluble substance, or

c) the volume of isopropanol being 10 to 30 V/V% of the aqueous liquid and the amount of lecithin being not more than 50 parts by weight per part by weight of the fat-soluble substance.

**2.** The aqueous liquid of claim 1 which is an injectable preparation, an orally administratable liquid preparation, a lotion or a hair tonic.

**3.** The aqueous liquid of claim 1 wherein the lecithin is selected from a phospholipid-containing substance extracted from egg yolk or from a vegetable oil and optionally purified; a hydrogenation product thereof; synthetic glycerophosphoric acid esters of the following formula; and mixtures thereof:

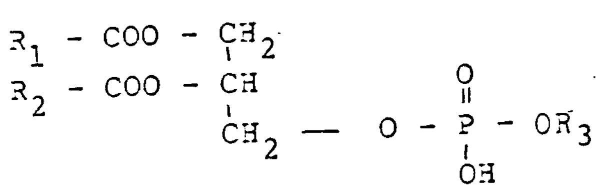

wherein $R_1$ and $R_2$ each represent hydrogen atom or a saturated or unsaturated hydrocarbon having 8 to 20 carbon atoms, and $R_3$ represents hydrogen atom, choline, ethanolamine, serine, inositol or

glycerol.

**Claims for the following Contracting State : AT**

**1.** A process for preparing an aqueous liquid containing as essential components a fat-soluble substance selected from vitamin A, vitamin E, vitamin D, vitamin K, ubidecarenone, derivatives of these substances, bisabolol, salicylic acid esters, squalane, isopropyl myristate and vegetable oils, furthermore lecithin and at least one alcohol selected from ethanol and isopropanol, wherein the respective components are mixed in the following ratios:

a) the volume of ethanol being not more than 50 V/V% of the aqueous liquid, and the amount of lecithin being less than 0.1 part by weight per part by weight of the fat-soluble substance, when ethanol is used as the alcohol in an amount of less than 50 parts by weight per part by weight of the fat-soluble substance, or

b) the volume of ethanol being not more than 50 V/V% of the aqueous liquid, and the amount of lecithin being not more than 50 parts by weight per part by weight of the fat-soluble substance, when ethanol is used as the alcohol in an amount of at least 50 parts by weight per part by weight of the fat-soluble substance, or

c) the volume of isopropanol being 10 to 30 V/V% of the aqueous liquid and the amount of lecithin being not more than 50 parts by weight per part by weight of the fat-soluble substance.

**2.** A process according to claim 1 wherein the aqueous liquid is an injectable preparation, an orally administratable liquid preparation, a lotion or a hair tonic.

**3.** A process according to claim 1 wherein the lecithin is selected from a phospholipid-containing substance extracted from egg yolk or from a vegetable oil and optionally purified; a hydrogenation product thereof; synthetic glycerophosphoric acid esters of the following formula; and mixtures thereof:

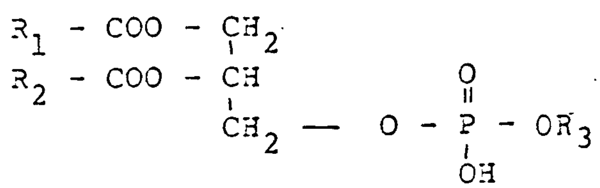

wherein $R_1$ and $R_2$ each represent hydrogen atom or a saturated or unsaturated hydrocarbon having 8 to 20 carbon atoms, and $R_3$ represents hydrogen atom, choline, ethanolamine, serine, inositol or glycerol.

**Patentansprüche**
**Patentansprüche für folgende Vertragsstaaten : BE, CH, DE, FR, GB, IT, LI, NL, SE**

**1.** Wäßrige Flüssigkeit, enthaltend als wesentliche Komponenten eine fettlösliche Substanz, ausgewählt aus Vitamin A, Vitamin E, Vitamin D, Vitamin K, Ubidecarenon, Derivaten dieser Substanzen, Bisabolol, Salicylsäureestern, Squalan, Isopropylmyristat und pflanzlichen Ölen, weiterhin Lecithin und mindestens einen Alkohol, ausgewählt aus Ethanol und Isopropanol, wobei

a) das Volumen an Ethanol nicht mehr als 50 V/V % der wäßrigen Flüssigkeit beträgt, und die Menge an Lecithin weniger als 0,1 Gew.-Teile pro Gew.-Teil der fettlöslichen Substanz beträgt, wenn Ethanol als der Alkohol in einer Menge von weniger als 50 Gew.-Teile pro Gew.-Teil der fettlöslichen Substanz verwendet wird, oder

b) das Volumen an Ethanol nicht mehr als 50 V/V % der wäßrigen Flüssigkeit beträgt, und die Menge an Lecithin nicht mehr als 50 Gew.-Teile pro Gew.-Teil der fettlöslichen Substanz ist, wenn Ethanol als der Alkohol in einer Menge von mindestens 50 Gew.-Teilen pro Gew.-Teil der fettlöslichen Substanz verwendet wird, oder

c) das Volumen an Isopropanol 10 bis 30 V/V % der wäßrigen Flüssigkeit beträgt, und die Menge an Lecithin nicht mehr als 50 Gew.-Teile pro Gew.-Teil der Fettlöslichen Substanz ist.

**2.** Wäßrige Flüssigkeit nach Anspruch 1, die eine injizierbare Zubereitung, eine oral verabreichbare, flüssige Zubereitung, eine Lotion oder ein Haarwasser ist.

**3.** Wäßrige Flüssigkeit nach Anspruch 1, wobei das Lecithin aus einer phospholipidhaltigen Substanz, extrahiert aus Eigelb oder einem pflanzlichen Öl und gegebenenfalls gereinigt, einem hydrierten Produkt hiervon, synthetischen Glycerophosphorsäureestern der folgenden Formel und Mischungen hiervon ausgewählt ist:

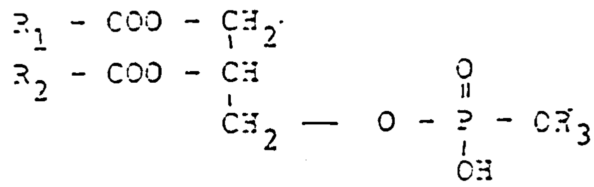

wobei $R_1$ und $R_2$ jeweils ein Wasserstoffatom oder eine gesättigte oder ungesättigte Kohlenwasserstoffgruppe mit 8 bis 20 Kohlenstoffatomen darstellen, und $R_3$ ein Wasserstoffatom, Cholin, Ethanolamin, Serin, Inosit oder Glycerin bedeutet.

**Patentansprüche für folgenden Vertragsstaat : AT**

**1.** Verfahren zur Herstellung einer wäßrigen Flüssigkeit, enthaltend als wesentliche Komponenten eine fettlösliche Substanz, ausgewählt aus Vitamin A, Vitamin E, Vitamin D, Vitamin K, Ubidecarenon, Derivaten dieser Substanzen, Bisabolol, Salicylsäureestern, Squalan, Isopropylmyristat und pflanzlichen Ölen, weiterhin Lecithin und mindestens einen Alkohol, ausgewählt aus Ethanol und Isopropanol, wobei die entsprechenden Komponenten in dem folgenden Verhältnis vermengt werden:

a) das Volumen an Ethanol beträgt nicht mehr als 50 V/V % der wäßrigen Flüssigkeit, und die Menge an Lecithin ist weniger als 0,1 Gew.-Teile pro Gew.-Teil der fettlöslichen Substanz, wenn Ethanol als der Alkohol in einer Menge von weniger als 50 Gew.-Teile pro Gew.-Teil der fettlöslichen Substanz verwendet wird, oder

b) das Volumen an Ethanol beträgt nicht mehr als 50 V/V % der wäßrigen Flüssigkeit, und die Menge an Lecithin beträgt nicht mehr als 50 Gew.-Teile pro Gew.-Teil der fettlöslichen Substanz, wenn Ethanol als der Alkohol in einer Menge von mindestens 50 Gew.-Teilen pro Gew.-Teil der fettlöslichen Substanz verwendet wird, oder

c) das Volumen an Isopropanol beträgt 10 bis 30 V/V % der wäßrigen Flüssigkeit, und die Menge an Lecithin ist nicht mehr als 50 Gew.-Teile pro Gew.-Teil der fettlöslichen Substanz.

**2.** Verfahren nach Anspruch 1, wobei die wäßrige Flüssigkeit eine injizierbare Zubereitung, eine oral verabreichbare, flüssige Zubereitung, eine Lotion oder ein Haarwasser ist.

**3.** Verfahren nach Anspruch 1, wobei das Lecithin aus einer phospholipidhaltigen Substanz, extrahiert aus Eigelb oder einem pflanzlichen Öl und gegebenenfalls gereinigt, einem hydrierten Produkt hiervon, synthetischen Glycerophosphorsäureestern der folgenden Formel und Mischungen hieraus ausgewählt wird:

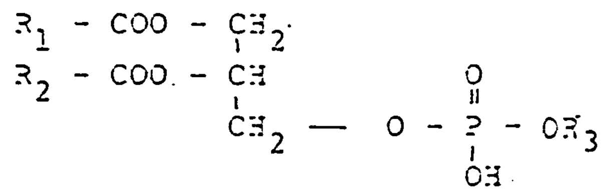

wobei $R_1$ und $R_2$ jeweils ein Wasserstoffatom oder eine gesättigte oder ungesättigte Kohlenwasserstoffgruppe mit 8 bis 20 Kohlenstoffatomen bedeuten, und $R_3$ ein Wasserstoffatom, Cholin, Ethanolamin, Serin, Inosit oder Glycerin ist.

## Revendications

**Revendications pour les Etats contractants suivants : BE, CH, DE, FR, GB, IT, LI, NL, SE**

1. Liquide aqueux contenant, comme constituants essentiels, une substance liposoluble choisie parmi la vitamine A, la vitamine E, la vitamine D, la vitamine K; l'ubidécarénone, les dérivés de ces substances, le bisabolol, les esters d'acide salicylique, le squalane, le myristate d'isopropyle et les huiles végétales, et aussi de la lécithine et au moins un alcool choisi parmi l'éthanol et l'isopropanol
   a) le volume d'éthanol ne dépassant pas 50% en vol/vol du liquide aqueux et la quantité de lécithine étant inférieure à 0,1 partie en poids par partie en poids de substance oléosoluble, si l'éthanol est utilisé comme alcool à raison de moins de 50 parties en poids par partie en poids de substance oléosoluble, ou
   b) le volume d'éthanol ne dépassant pas 50% en vol/vol du liquide aqueux et la quantité de lécithine ne dépassant pas 50 parties en poids par partie en poids de substance oléosoluble, si l'éthanol est utilisé comme alcool à raison d'au moins 50 parties en poids par partie en poids de substance oléosoluble, ou
   c) le volume d'isopropanol étant de 10 à 30% en vol/vol du liquide aqueux et la quantité de lécithine n'étant pas supérieure à 50 parties en poids par partie en poids de substance oléosoluble.

2. Liquide aqueux selon la revendication 1, qui est une préparation injectable, une préparation liquide pour administration orale, une lotion ou un tonique capillaire.

3. Liquide aqueux selon la revendication 1, dans lequel la lécithine est choisie parmi une substance contenant un phospholipide extraite du jaune d'oeuf ou d'une huile végétale et éventuellement purifiée; un produit d'hydrogénation de cette dernière; des esters d'acide glycérophosphorique synthétiques de formule suivante; et leurs mélanges;

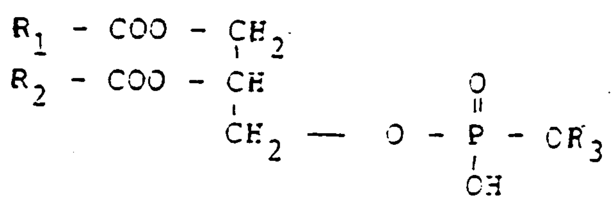

où $R_1$ et $R_2$ représentent chacun un atome d'hydrogène ou un hydrocarbure saturé ou insaturé comportant 8 à 20 atomes de carbone, et $R_3$ représente un atome d'hydrogène, la choline, l'éthanolamine, la sérine, l'inositol ou le glycérol.

**Revendications pour l'Etat contractant suivant : AT**

1. Procédé de préparation d'un liquide aqueux contenant, comme constituants essentiels, une substance liposoluble choisie parmi la vitamine A, la vitamine E, la vitamine D, la vitamine K; l'ubidécarénone, les dérivés de ces substances, le bisabolol, les esters d'acide salicylique, le squalane, le myristate d'isopropyle et les huiles végérales, et aussi de la lécithine et au moins un alcool choisi parmi l'éthanol et l'isopropanol, dans lequel les constituants respectifs sont mélangés dans les proportions suivantes :
   a) le volume d'éthanol ne dépassant pas 50% en vol/vol du liquide aqueux et la quantité de lécithine étant inférieure à 0,1 partie en poids par partie en poids de substance oléosoluble, si l'éthanol est utilisé comme alcool à raison de moins de 50 parties en poids par partie en poids de substance oléosoluble, ou
   b) le volume d'éthanol ne dépassant pas 50% en vol/vol du liquide aqueux et la quantité de lécithine ne dépassant pas 50 parties en poids par partie en poids de substance oléosoluble, si l'éthanol est utilisé comme alcool à raison d'au moins 50 parties en poids par partie en poids de substance oléosoluble, ou
   c) le volume d'isopropanol étant de 10 à 30% en vol/vol du liquide aqueux et la quantité de lécithine n'étant pas supérieure à 50 parties en poids par partie en poids de substance oléosoluble.

2. Procédé selon la revendication 1, dans lequel le liquide aqueux est une préparation injectable, une préparation liquide pour administration orale, une lotion ou un tonique capillaire.

**3.** Procédé selon la revendication 1, dans lequel la lécithine est choisie parmi une substance contenant un phospholipide extraite du jaune d'oeuf ou d'une huile végétale et éventuellement purifiée; un produit d'hydrogénation de cette dernière; des esters d'acide glycérophosphorique synthétiques de formule suivante; et leurs mélanges;

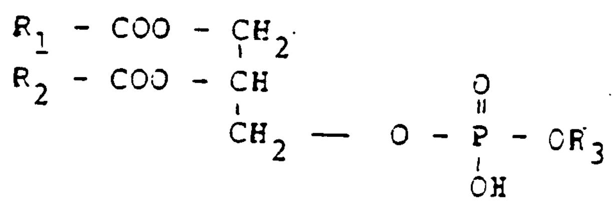

où $R_1$ et $R_2$ représentent chacun un atome d'hydrogène ou un hydrocarbure saturé ou insaturé comportant 8 à 20 atomes de carbone, et $R_3$ représente un atome d'hydrogène, la choline, l'éthanolamine, la sérine, l'inositol ou le glycérol.